(19) **Europäisches Patentamt / European Patent Office / Office européen des brevets**

(11) **EP 1 831 681 B1**

(12) **EUROPEAN PATENT SPECIFICATION**

(45) Date of publication and mention
of the grant of the patent:
**13.10.2010 Bulletin 2010/41**

(21) Application number: **04806906.6**

(22) Date of filing: **30.12.2004**

(51) Int Cl.:
*G01N 33/00* (2006.01)   *G01N 27/12* (2006.01)

(86) International application number:
**PCT/IT2004/000743**

(87) International publication number:
**WO 2006/070420 (06.07.2006 Gazette 2006/27)**

(54) **METHOD FOR THE CONTROL OF THE RESPONSE TIME OF A SENSOR FOR CHEMICAL SUBSTANCES**

VERFAHREN ZUR STEUERUNG DER REAKTIONSZEIT EINES SENSORS FÜR CHEMISCHE SUBSTANZEN

PROCEDE SERVANT A CONTROLER LE TEMPS DE REACTION D'UN DETECTEUR DE SUBSTANCES CHIMIQUES

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**12.09.2007 Bulletin 2007/37**

(73) Proprietor: **Universita' degli Studi di Roma " Tor Vergata"**
**00173 Roma (IT)**

(72) Inventors:
• **D'AMICO, Arnaldo**
  **I-00178 Roma (IT)**
• **DI CARLO, Aldo**
  **I-00177 Roma (IT)**
• **DI NATALE, Corrado**
  **I-00177 Roma (IT)**
• **LUCCI, Massimiliano**
  **I-00155 Roma (IT)**
• **PAOLESSE, Roberto**
  **I-00175 Roma (IT)**
• **REALE, Andrea**
  **I-00177 Roma (IT)**
• **TERRANOVA, Maria Letizia**
  **I-00157 Roma (IT)**

(74) Representative: **Iannone, Carlo Luigi et al**
**Barzanò & Zanardo Roma S.p.A.**
**Via Piemonte 26**
**00187 Roma (IT)**

(56) References cited:
**EP-A- 0 425 119    DE-A1- 4 442 396**

• **J. SUEHIRO ET AL.: "Fabrication of a carbon nanotube-based gas sensor using dielectrophoresis and its application for ammonia detection by impedance spectroscopy" JOURNAL OF PHYSICS D: APPLIED PHYSICS, vol. 36, 2003, pages L109-L114, XP002342595**
• **VARGHESE O K ET AL: "Gas sensing characteristics of multi-wall carbon nanotubes" SENSORS AND ACTUATORS B, ELSEVIER SEQUOIA S.A., LAUSANNE, CH, vol. 81, no. 1, 15 December 2001 (2001-12-15), pages 32-41, XP004324017 ISSN: 0925-4005**
• **VALENTINI L ET AL: "Sensors for sub-ppm NO2 gas detection based on carbon nanotube thin films" APPLIED PHYSICS LETTERS, AMERICAN INSTITUTE OF PHYSICS. NEW YORK, US, vol. 82, no. 6, 10 February 2003 (2003-02-10), pages 961-963, XP012034767 ISSN: 0003-6951**

## Description

The present invention concerns a method for the control of the response time of a sensor for chemical substances.

More in detail the invention pertains to a method of control of a sensor that foresees active substances that through the contact with determined gas change their own electric characteristics, for instance the impedance. Said method, being able to drastically reduce the times of detection/recovery, maintaining the characteristics of the sensor of an elevated sensibility, a low dependence of the sensibility in comparison to the temperature, and having been further studied and realized particularly for the detection of nitrogen dioxide $NO_2$ or ammonia $NH_3$), but that it can be used for any other gas.

In the following the description will be addressed to the detection of ammonia or nitrogen dioxide, but it is well evident that the description itself does not have to be considered as limited to this specific use.

As it is well known, the sensors of gaseous substances generally use some materials sensitive to the gases to be detected. Detection can occur through different chemical-physical phenomena's. Among these, the variation of impedance of the material is currently object of particular attention. In fact, it has been noted that semiconductor materials or nanotubes layers show this interesting phenomenon.

As far as nanotubes, and particularly the carbon nanotubes, are concerned, since their discovery, have aroused a lot of interest in the scientific community, due to their electronic and mechanical characteristics.

Currently the carbon nanotubes are object of studies especially in the area of electronics, biosensors, medicine and realization of chemical sensors. With reference to this last application, it has been noted that they provides a series of advantages in comparison to other materials, among which:

- high sensitivity;
- low dependence of the sensitivity with respect to the temperature;
- linearity of the response also for low concentrations.

The nanotubes consist in folded graphite sheets. They have a diameter of the order of few nanometres (nm) and a length also superior to $100\mu m$. Substantially they form very thin interconnection wires.

The realization of said nanotubes occurs by the wrapping of monoatomic layers of graphite sheets along one set direction. Said direction determines both the diameter and the chirality. These variables determine the nature of semiconductor or conductor of the layer.

As already said, currently, it is very promising the use of nanotubes in sensors for gas detection.

It is known, in fact, that the doping of the carbon nanotubes brings to sensitive variations of the conductance, also for concentrations of gas of some hundred parts per million (ppm) of the same gas.

Another design variable related to the nanotubes is the fact that they can be deposited in a random or ordered way. Sensitivity generally results best in the case in which nanotubes are used deposited in ordered way, because they introduce a greater effective surface.

As already said, sensors with nanotubes are very sensitive. One common technical problem for many gas sensor devices based on the variation of impedance, concerns to the slow recovery times of the same sensor. In other words, following an experimental measure, the molecules of the gas the presence of which is to be detected, remains adherent for a lot of time to the walls of the carbon nanotubes, or of the active material of the sensor in general. It must be taken into consideration that the recovery time generally results to be, during the experiment, of the order of many tenths of minutes.

A technique currently used for reducing the desorption times of the molecules from the sensor is to radiate said carbon nanotubes layers by light. Particularly, ultraviolet light is used.

This system, despite is very efficient, is poorly applicable for the realization of sensors suitable for the marketing, because it is very expensive and complex at the same time. Finally, engineering could not be able to allow the same performances in the desorption times.

Some techniques currently exist providing the application of a constant electric field on the surface of the active material, so as to facilitate the desorption of the detected molecules. This system revealed to be rather efficient. However, the problem of the reduction of the absorption time has not been resolved as yet, this problem being the most important from the application view. It is, in fact, obvious that as primary function the sensor has to be able to perceive a danger in the shortest possible time, before being able to make another measure.

It appears, therefore, evident that the technical problem of the absorption/desorption times is very important for sensors, mainly to increase the probabilities of a commercial success.

An example of prior art is given by the patent application DE 4442396 A1, which discloses a gas sensor having a thin layer of silicon to hold a field electrode of precious metal or polycrystalline silicon below a sensor layer carrying interdigital electrodes. Said field electrode is insulated from the substrate by a layer of silicon dioxide or silicon nitride and from the sensor by another oxide layer. To achieve an even passage of the electrical field across the sensor, a second silicon substrate carries a metallised electrode above the sensor. This is attached to the first substrate by means of the "flip-chip" technique or by electrically conductive adhesive. In this way the distance between the field electrodes and thus the air gap is minimized. By application of bias voltages gas desorption is quickly measured.

EP 425119 A1, which again forms part of the state of the art, describes an apparatus and method for determining the concentration of a pollutant gas such as NOx or CO/CH4 in a gaseous atmosphere such as ambient air.

Therefore, the object of the present invention is to propose a sensor whose response times, both for the absorption and for the desorption of the molecules to be detected, can be easily controlled, allowing remarkable reductions of the same times.

Another object of the invention is that of obtaining such control through an electronic device or in general through the use of an electric signal.

Further object of the present invention is that of allowing to detect the composition of the mixture of air to which the sensor is subjected.

It is therefore specific object of the present invention a method for the control of the response time of a sensor for chemical substances, said sensor being comprised of a first and a second pins, at least a layer of active material deposited between said first and said second pins, said active material varying its own impedance following the contact with said chemical substances and as a function of their concentration, and a further control pin; said method being **characterized in that** it provides at least a phase of detection of said chemical substances corresponding to an absorption of said chemical substances by said material, and at least a phase of recovery of said sensor, corresponding to a desorption of said chemical substances by said material; and in that during said phases of detection and recovery a signal is applied to said control pin, suited to solicit the absorption or desorption of said material varying the duration of the respective phases of detection and recovery.

Always according to the invention, said signal can provide a component in (DC) direct current so as:

- to negatively polarize said control pin with respect to the active material in the phase of absorption, in case the molecule of the gas to be detected allows a joining with said active material of the electron donor type, or positively in case the molecule of the gas to be detected allows a joining with said active material of the electron withdrawer type;
- to positively polarize said control pin with respect to the active material in the phase of desorption, in case the molecule of the gas to be detected allows a joining with said active material of the electron donor type, or negatively in case the molecule of the gas to be detected allows a joining with active said material of the electron withdrawer type.

Furthermore, according to the invention, said signal can provide a frequency component, that can vary between 0 Hz and 100 KHz, suited to solicit the molecules of said gaseous kinds and/or to be greater than 100 KHz, so as to interact on the bond among said active material and said gaseous kinds.

Preferably according to the invention, said signal can have a sinusoidal waveform, a quadratic waveform or a pulsed waveform of arbitrary form.

Always according to the invention, said active material can be comprised of nanostructurated materials, preferably carbon nanotubes, deposited between said first and said second pins.

Advantageously, according to the invention, said nanotubes can be of the single wall type and deposited in an ordered way, preferably by dielectrophoresys.

Further, according to the invention, said active material can be comprised of metallic oxides deposited between said first and said second pins, that can be tin oxide ($SnO_2$) and/or tungsten oxide ($WO_3$).

Still according to the invention, said active material can be comprised of organic semiconductor materials and organic-metallic materials deposited between said first and said second pins.

Advantageously, according to the invention, said sensor can include an insulating layer, above which said first and said second pins are placed, and beneath which said control pin is placed.

Still according to the invention, said first and said second pins can be interdigitated and said insulating layer can be comprised of silicon dioxide ($SiO_2$) or glass.

Advantageously according to the invention, said chemical substances can include nitrogen dioxide $NO_2$ or ammonia $NH_3$.

There is also herein described an example of a method for the detection of the composition of a mixture of gas, **characterized in that** it comprises a sensor of chemical substances, said sensor being polarized in such way that a current flows through the active material, said method being further **characterized in that** it provides the following phases:

a. detecting the spectrum of the output signal of one of said first or second pins with respect to said control pin;
b. extracting from said spectrum one or more measures;
c. comparing said one or more measures obtained with reference measures in order to detect the presence of a kind of gas.

Always according to the invention, said sensor provides said first pin and it can be connected to a reference potential, said second pin connected to a greater potential with respect to said reference potential by a resistive dipole and said control pin to a signal generator.

Still according to the invention, said measures can include the detection of the cut off frequency and/or the detection of the depths of the resonance curves and/or the detection of the resonance frequencies.

The present invention will be now described, for illustrative but not limitative purposes, according to its preferred embodiments, with particular reference to the figures of the enclosed drawings, wherein:

figure 1 shows a top section view of the sensor for chemical substances with ordered single wall carbon nanotubes;

figure 2 shows a side section view of the sensor according to the figure 1;

figure 3 shows a graph of a lateral view of a model of interaction of a nanotube with a gaseous molecule;

figure 4 shows an experimental model for the analysis of the working parameters of the sensor according to the present invention;

figure 5 shows the graph of the sensitivity of the sensor according to figure 1 as a function of the ammonia concentration for different static voltage (DC) applied on the gate;

figure 6 shows the graph of the sensitivity of the sensor according to figure 1 as a function of the static voltage (DC) applied on the gate;

figure 7 shows the graph of the sensitivity of the sensor according to figure 1 as a function of the elapsed time for different ammonia concentrations and different static voltage (DC) applied on the gate; figure 8 shows the graph of the sensitivity of the sensor according to figure 1 and of the concentration of ammonia as a function of the elapsed time, with respect to pulsed gate voltage during the desorption phase;

figure 9 shows the graph of the sensitivity of the sensor according to figure 1 and of the concentration of ammonia as a function of the elapsed time, with respect to pulsed gate voltage during the absorption and desorption phases;

figure 10a shows the graph of the sensitivity for a sensor according to figure 1 as a function of the ammonia concentration, with respect to two different temperatures;

figure 10b shows the graph of the sensitivity for a sensor according to figure 1 as a function of the temperature;

figure 11a shows the graph of the sensitivity for a sensor with ordered single wall carbon nanotubes and for a sensor with disordered single wall carbon nanotubes as a function of the ammonia concentration;

figure 11b shows the graph of the sensitivity for a sensor with ordered single wall carbon nanotubes and for a sensor with disordered single wall carbon nanotubes;

figure 12 shows an electric scheme in which the sensor is assembled according to figure 1; and

figure 13 shows the graph of the Bode diagram of the amplitude-frequency characteristic of a sensor according to figure 1.

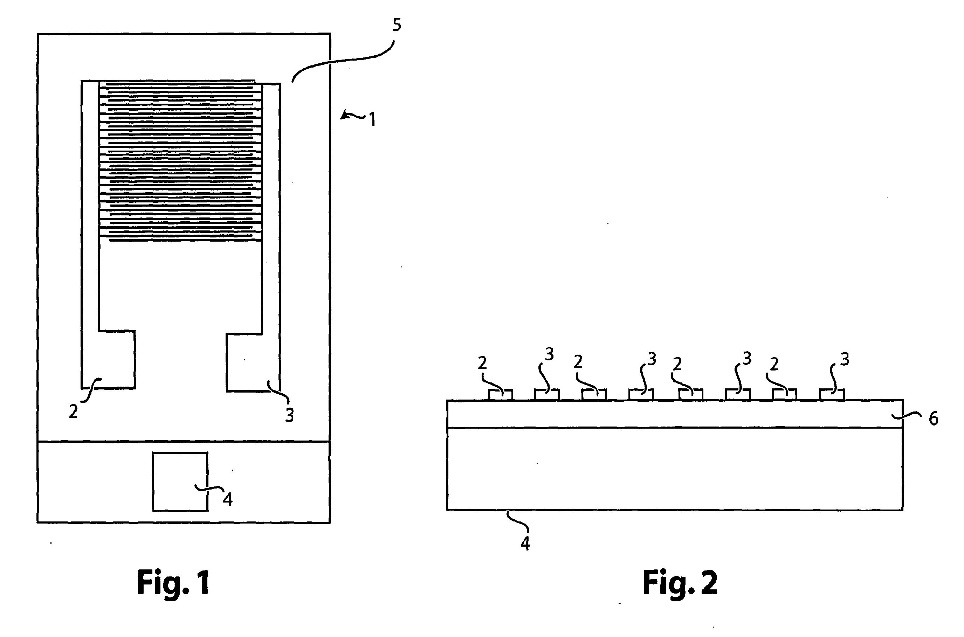

Making reference to figure 1, it is possible to observe the sensor 1 in a top section view. Said sensor 1 includes three pins. A first source pin 2, a second drain pin 3 and one control or gate pin 4.

The source 2 and drain 3 pins are interdigitated on the surface 5. On said pins are further deposited carbon nanotubes that, following the interaction with a molecule of a gas to be detected, vary their impedance.

Therefore, the assembly comprised of the source 2 and drain 3 pins and carbon nanotubes as active material, substantially forms a variable resistor as a function of the concentration of gas to which said material is submitted.

the gate pin 4 extends itself under the surface 5. By a suitable signal, on the gate 4, it is possible to control, i.e. to reduce the absorption and/or desorption time of said gas to be detected, allowing to obtain with greater rapidity the necessary information relevant to the presence of the gas, or to make more quickly reusable the sensor 1 for a new measure.

Observing figure 2, it is possible to analyse in greater detail the structure of sensor 1, particularly with respect to the surface 5. In fact, it is possible to observe the source 2 and drain 3 pins interdigitated and the underlying gate 4, realized in silicon (Si).

Between the gate 4 and the surface 5 it is present an insulating layer 6 that in this embodiment is comprised of silicon dioxide $SiO_2$.

On the sources 2 and drain 3 pins, nanotubes of carbon 7 are deposited. These are single-walled nanotubes. This allows a greater sensitivity of the sensor 1. Nevertheless, this is not a limitative characteristic of the invention, since it could be also possible using other nanocomposite of carbon or other materials, as for instance multi walled carbon nanotubes, that as known are comprised of concentric cylinders, surfaces with nano-diamond or metallic oxides and/or organic semiconductors.

In the present example of sensor 1, carbon nanotubes 7 are deposited in an ordered way. Also this characteristic is not limitative for the present invention.

In this example, the deposition method for the layer of carbon nanotubes 7 to obtain its alignment makes use of the well known technique of dielectrophoresys. Said technique consists in the fact that when a generic dielectric particle is placed in an electric field it experiences the action of a dielectrophoretic force.

In the present example, single wall carbon nanotubes are first synthesized by arc discharge technique, subsequently purified using $HNO_3$ and finally dispersed in Chloroform and Dimethylformamide.

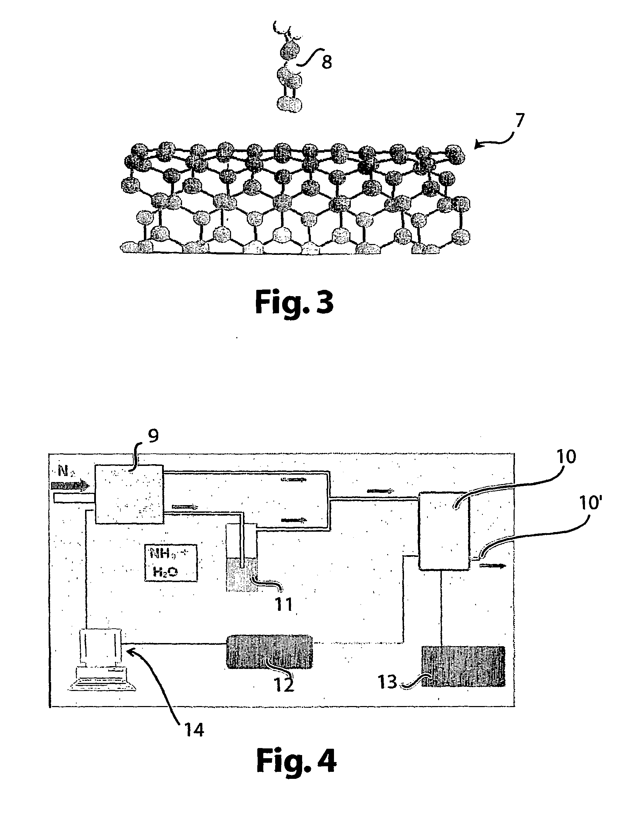

In figure 3 it is possible observing the interaction between a carbon nanotube 7 and a molecule of a gas 8. Following the interaction with a molecule of $NO_2$ or $NH_3$, for instance, it occurs a variation of the impedance of the nanotube 7. The reason of such phenomenon is generally attributed to a mechanism of charge transfer. In other words, some gaseous kinds are able to alter the impedance of the carbon nanotubes 7 following the exchange of charge carriers. In the case under examination, molecules of $NH_3$ assume the form of electron donor, while those of $NO_2$ of electron withdrawer.

By a suitable signal on the gate 4 it is possible to increase the coupling, and therefore the absorption or the uncoupling, and therefore the desorption, of the gaseous molecules. The characteristics of said signal to get the aforesaid phenomenon are:

• polarity: depending on the fact if the gas to be detected is electron donor or electron withdrawer, with a suitable choice of the polarity of the voltage on the gate 4, and therefore of the direction of the electric field with respect to the perpendicular to said surface 5, an electrostatic repulsion or electrostatic attraction will occur that will favour the coupling or uncoupling of the molecules to the surface of the nanotubes 7;

• amplitude of the signal: this is proportional to the intensity of the electric field on the surface 5. Therefore, the greater is the intensity of the voltage on the gate 4, the greater is the energy of the electric field described in the preceding point;

• frequency: the second mechanism by which a coupling or uncoupling of the molecule is that of subjecting carbon nanotubes 7 to an electric field that has a frequency able to condition the bond between the molecule of the gas and the surface of the carbon nanotubes 7. Efficiency of such interaction can depend on the frequency of the applied electric field. Particularly, conditions of resonance can be obtained depending on the specific bond energy between gas and active substance. However, also the application of a low frequency signal stimulates in an effective way the molecules.

With such systems it is even possible to obtain a reduction of two orders of magnitude of the absorption or desorption times for the gas.

For the detection of the performances of sensor 1 according to the present invention, an experimental apparatus has been used as shown in figure 4. In the case under examination, said apparatus is prepared for the detection of the ammonia ($NH_3$) molecules.

In the apparatus it is possible to observe that a nitrogen ($N_2$) flux enters a device controlling the flux 9. Part of said nitrogen flux enters the cell 10, said cell 10 providing an exit 10' of the gas, in which the sensor 1 is contained, and a part is sent within a bubbler 11, at the outlet being obtained the ammonia vapour $NH_3$ which is sent within the cell 10.

A multimeter 12 and a temperature controller and sensor 13 are connected to said cell 10. Finally, a processing unit 14 controls both the multimeter 12 and flux controller 9.

To evaluate the performances of sensor 1 according to the present invention, in the following some experimental data are shown. In order to make clearer the features of sensor 1, the electrostatic effects will be analysed, these effects being obtained by the application of a constant voltage of gate 4 in the absorption/desorption phases. Then, a frequency signal will be added to the signal of the polarized gate 4.

The data are shown for the detection of ammonia ($NH_3$), that, it must be remembered, when bond with a nanotube operates like a donor molecule. Particularly, the typical measured parameter is the relative resistance, defined as:

$$\rho(c) = \left.\frac{\Delta R}{R_0}\right|_c$$

being c the concentration of the detected substance. The slope of p expresses the sensitivity of the sensor in ($\Delta R/R$)/ppm.

*Electrostatic analysis*

A sensor with ordered single wall carbon nanotubes carries out experimental analyses.

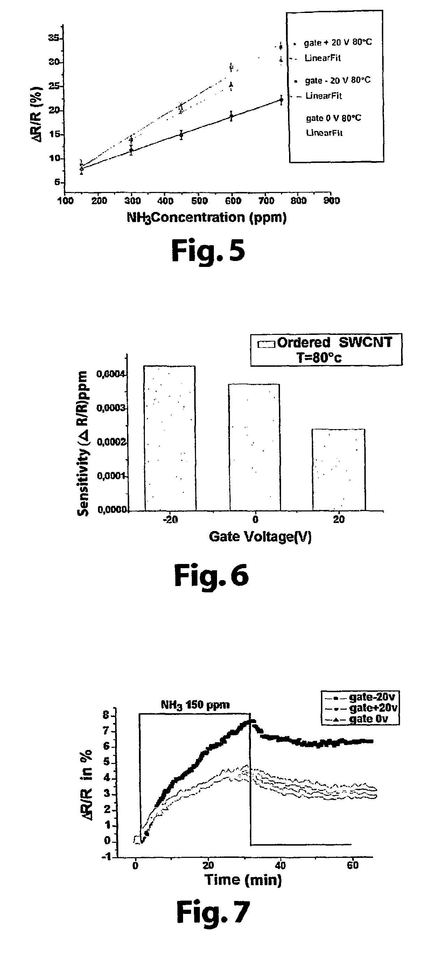

Figures 5 and 6 show the behaviour of the sensitivity, i.e. of the relative resistance p, obtained by varying the gate voltage 4 for five different concentrations of $NH_3$. It is demonstrated how the interaction among carbon nanotubes can be controlled acting by an electrostatic way, polarizing the gate 4. Varying said voltage among +20Volt and -20Volt, the sensitivity changes of about 100%. It is further evident the linearity of the characteristic.

In figure 7 it is observed how the gate voltage 4 applied to the substrate also influences the time control of the sensor characteristic. In fact, in the case in which a negative voltage is applied to the gate 4, ammonia (electron donor) interacts more easily (and quickly). This is confirmed by the fact that, as it can be seen, the corresponding signal has a greater average slope.

In general, it is possible using the following scheme for the phenomena of electrostatic absorption/desorption:

• type of molecule: electron donor (e.g. $NH_3$);

   - polarization voltage of the gate for improvement of the absorption time: negative;
   - polarization voltage of the gate for improvement of the desorption time: positive;

• electron withdrawer (e.g. $NO_2$);

   - polarization voltage of the gate for improvement of the absorption time: positive;
   - polarization voltage of the gate for improvement of the desorption time: negative;

The use of a gate voltage 4 also solves a further problem.

Sensors 1 realized with single wall carbon nanotubes can introduce a problem when they are subjected to a mixture of gas, either of electron-donor type, either of electron-withdrawer type. In fact, relationship of concentration between the two gases could be at a particular value, so that it does not make varying the resistivity of the sensor. This is due to the fact that the single gases have the tendency to vary the resitivity in an opposite way, thus balancing their effect.

By the use of opposite gate voltages 4, it is possible to promote the absorption of one or the other gaseous kind detecting both and overcoming said problem.

*Analysis in frequency*

Always using the same kind of sensor above mentioned, gate voltages 4 are applied with positive or negative medium value, so as to obtain the above described electrostatic effects, but an oscillating signal is added, in the present case a quadratic signal, with frequency of about 1 KHz.

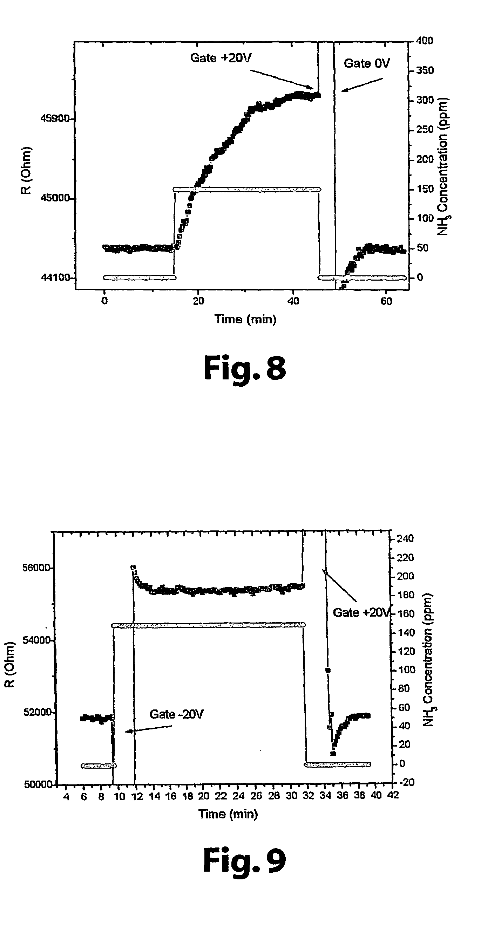

Figure 8 shows, particularly, the effect of the improvement of the sensor response due to the application of a gate voltage 4 of 0Volt in the phase of absorption and of +20Vpp for three minutes in the phase of desorption. It can be noted that the molecules of the gas instantly desorbs.

The same technique can be used for the improvement of the response time in the absorption phase, as shown in figure 9. In this case:

- voltage of gate 4 in the absorption phase equal to -20Vpp, applied for 3 minutes;
- voltage of gate 4 in the desorption phase equal to +20Vpp, applied for 3 minutes.

As it is deduced, both phases are strongly improved.

The low frequency *range* (up to 100KHz) is considered interesting because it acts on the electrostatic interaction between sensor and molecules. The low frequency modulation, in fact, is useful because kinetically the transition on-off, greater in such frequency excursions, contributes to stimulate in a more effective way the molecules.

The high frequency *range* (from 100KHz) is useful because, as already said, it exploits the resonance features of the interaction of the various gaseous substances with the sensible materials, in the specific case with single wall carbon nanotubes, particularly it interacts with respect to the bond energies. One gets therefore a high selectivity of the response.

Materials that it is possible to use for the application of signals on the gate 4 for the improvement of the response time, besides the single or multiple wall carbon nanotubes, are many. Some of these can be identified in metallic oxides materials, particularly $SnO_2$ (tin oxide) and $WO_3$ (tungsten oxide), organic semiconductor materials and metallic-organic materials and other materials.

Always taking into consideration the nanotubes properties, it is evident from the analysis of graphs 10a and 10b how the sensitivity of carbon nanotubes sensors has a low dependence from the temperature. With the same gate voltage 4 (0Volt), and for an excursion of temperature from 23°C to 80°C, a variation of p of 6%-7% is obtained.

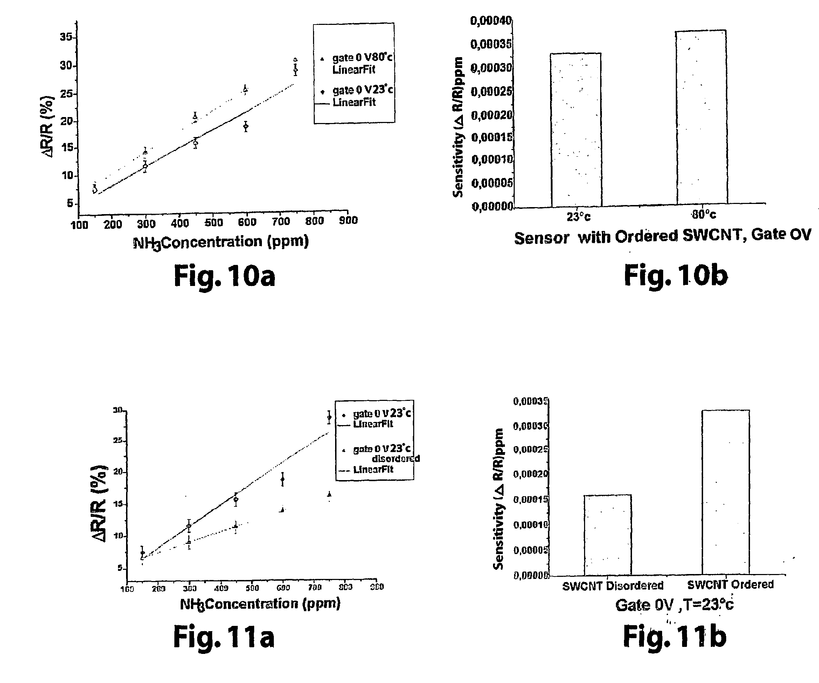

Finally, figures 11 a and 11 b show how sensors with ordered carbon nanotubes are more sensitive with respect to those in which nanotubes are randomly deposited.

Particularly, it is even observed a double sensitivity, being constant the other physical conditions.

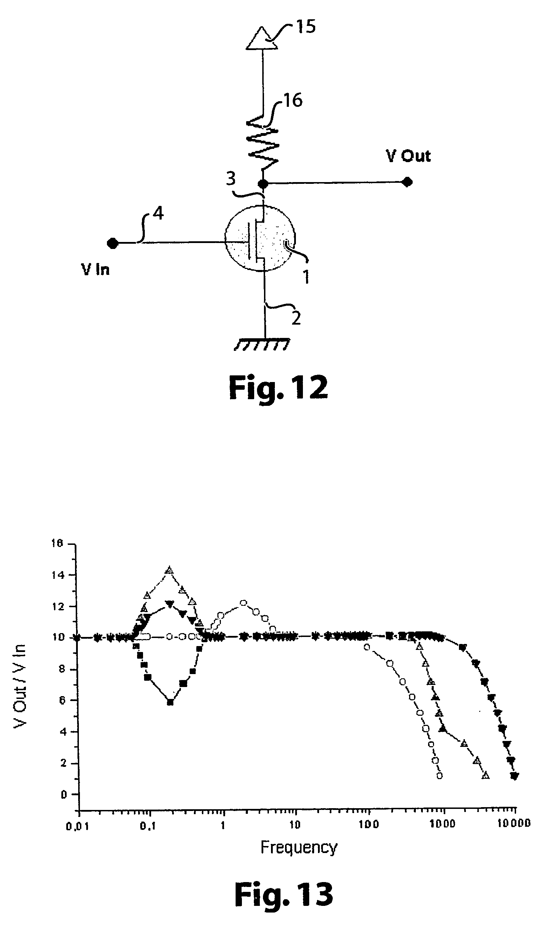

Sensor 1 can also be assembled in "amplifier configuration", as shown in the figure 12. Particularly, in the embodiment shown in the figure a "common source" configuration is observed, in which source 2 is connected to ground, drain is connected to a power supply 15 through a resistance 16. On drain 3 it is read the output signal, while on gate 4 frequency variable signals are applied.

By this configuration of the sensor and of the control gate 4, it is possible to consider how the interaction of sensor 1 with the various gases modifies the form of the Bode diagram (amplitude-frequency curve), an example of which can be seen in figure 13 (relationship between the output voltage on drain 3 and the input voltage on gate 4).

Particularly, from the analysis of said figure 13 it is possible observing the overlapping of four different gaseous substances. The frequency responses of said gaseous substances, obtained through the interaction with sensor 1, allows controlling the possible resonance frequencies and the cut off frequencies.

What is more remarkable of such application is the possibility of analysing how the different concentrations of gas, pure or mixed, modify this spectrum, for instance varying said cut off frequency or the depths of the curve for the same resonance frequency.

From the above description, it can be observed that the fundamental characteristic of the present invention is the possibility of reduction of the response times of a sensor with an active material varying its impedance as a function of the concentration of gas with which it interacts.

An advantage of the present invention is the fact that the effect of improvement of the response times can be obtained by the simple application of a suitable gate voltage that allows to drastically improve the response times of the sensor, making it usable in many fields. Said voltage can be applied in various ways, i.e. continuous, alternate to low frequency and alternate to high frequency according to the gaseous substance to be detected.

A further advantage of the present invention is the fact that the carbon nanotubes have shown very good behaviour for the detection of some gaseous substance, particularly Ammonia, $NO_X$ and gas nerve agent.

The present invention has been described for illustrative but not limitative purposes, according to its preferred embodiments, but it is to be understood that modifications and/or changes can be introduced by those skilled in the art without departing from the relevant scope as defined in the enclosed claims.

**Claims**

1.  Method for the control of the response time of a sensor (1) for chemical substances, said sensor (1) being comprised of
    a first (2) and a second (3) pins,
    at least a layer of active material deposited between said first (2) and said second (3) pins, said active material varying its own impedance following the contact with said chemical substances and as a function of their concentration, and
    a further control pin (4);
    said method provides
    at least a phase of detection of said chemical substances corresponding to an absorption of said chemical substances by said material, and
    at least a phase of recovery of said sensor (1), corresponding to a desorption of said chemical substances by said material,
    during said phases of detection and recovery, a signal being applied to said control pin (4), suited to solicit the absorption or the desorption of said material, varying the duration of the respective phases of detection and recovery;
    **characterized in that**
    said signal provides a component in (DC) direct current so as:

    - to negatively polarize said control pin (4) with respect to the active material in the phase of absorption, in case the molecule of the gas to be detected allows a joining with said active material of the electron donor type, or positively in case the molecule of the gas to be detected allows a joining with said active material of the electron withdrawer type;
    - to positively polarize said control pin (4) with respect to the active material in the phase of desorption, in case the molecule of the gas to be detected allows a joining with said active material of the electron donor type, or negatively in case the molecule of the gas to be detected allows a joining with active said material of the electron withdrawer type.

2.  Method according to claim 1, **characterized in that** said signal provides a frequency component.

3.  Method according to claim 2, **characterized in that** said signal has a frequency between 0 Hz and 100 KHz, suited to solicit the molecules of said gaseous kinds (8).

4.  Method according to claim 2 or 3, **characterized in that** said signal has a frequency greater than 100 KHz, so as to interact on the bond among said active material and said gaseous kinds.

5.  Method according to one of claims 2 - 4, **characterized in that** said signal has a sinusoidal waveform, a quadratic waveform.

6.  Method according to one of claims 2 - 4, **characterized in that** said signal has a pulsed waveform of arbitrary form.

7.  Method according to one of the preceding claims, **characterized in that** said active material is comprised of nanostructurated materials deposited between said first (2) and said second (3) pins, said nanostructurated materials comprising nanotubes.

8.  Method according to claim 7, **characterized in that** said nanotubes are carbon nanotubes (7).

9.  Method according to claim 8, **characterized in that** said nanotubes are of the single wall type.

10. Method according to one of claims 7 - 9, **characterized in that** said nanotubes are deposited in an ordered way, preferably by dielectrophoresis.

11. Method according to one of the preceding claims, **characterized in that** said active material is comprised of metallic oxides deposited between said first and said second pins.

12. Method according to claim 11, **characterized in that** said metallic oxides are tin oxide (Sn02) and/or tungsten oxide (W03).

13. Method according to one of the preceding claims, **characterized in that** said active material is comprised of organic semiconductor materials and organic-metallic materials deposited between said first (2) and said second (3) pins.

14. Method according to one of the preceding claims, **characterized in that** said sensor includes an insulating layer (6), above which said first (2) and said second (3) pins are placed, and beneath which said control pin is placed.

15. Method according to claim 14, **characterized in that** said insulating layer (6) is comprised of silicon dioxide (Si02) or glass.

16. Method according to one of the preceding claims, **characterized in that** said first (2) and said second (3) pins are interdigitated.

17. Method according to one of the preceding claims, **characterized in that** said chemical substances include nitrogen dioxide N02 or ammonia NH3.

**Patentansprüche**

1. Verfahren zur Steuerung der Reaktionszeit eines Sensors (1) für chemische Substanzen, wobei der Sensor (1) umfasst
erste (2) und zweite (3) Anschlüsse,
mindestens eine Schicht eines aktiven Materials, das zwischen den ersten (2) und zweiten (3) Anschlüssen abgelagert ist, wobei das aktive Material auf den Kontakt mit den chemischen Substanzen folgend und in Abhängigkeit von deren Konzentration seine eigene Impedanz variiert, und
ein weiterer Kontroll-Anschluss (4);
wobei das Verfahren bereitstellt
mindestens eine Phase der Detektion der chemischen Substanzen entsprechend einer Absorption der chemischen Substanzen durch das Material, und mindestens eine Phase der Regeneration des Sensors (1) entsprechend einer Desorption der chemischen Substanzen durch das Material, wobei während der Phasen der Detektion und Regeneration ein Signal an den Kontroll-Anschluss (4) angelegt wird, das geeignet ist, die Absorption oder die Desorption des Materials zu bewerten, wobei die Dauer der entsprechenden Phasen der Detektion und Regeneration variiert wird;
**dadurch gekennzeichnet, dass**
das Signal eine Komponente in Gleichstrom (DC) bereitstellt, sodass

   - in der Phase der Absorption der Kontroll-Anschluss (4) in Bezug auf das aktive Material, falls das Molekül des zu detektierenden Gases eine Verbindung mit dem aktiven Material des Elektron-Donor-Typs erlaubt, negativ polarisiert wird, oder, falls das Molekül des zu detektierenden Gases eine Verbindung mit dem aktiven Material des Elektron-Rückzugs-Typs erlaubt, positiv polarisiert wird; und
   - in der Phase der Desorption der Kontroll-Anschluss (4) in Bezug auf das aktive Material, falls das Molekül des zu detektierenden Gases eine Verbindung mit dem aktiven Material des Elektron-Donor-Typs erlaubt, positiv polarisiert wird, oder, falls das Molekül des zu detektierenden Gases eine Verbindung mit dem aktiven Material des Elektron-Rückzugs-Typs erlaubt, negativ polarisiert wird.

2. Verfahren gemäß Anspruch 1, **dadurch gekennzeichnet, dass** das Signal eine Frequenzkomponente bereitstellt.

3. Verfahren gemäß Anspruch 2, **dadurch gekennzeichnet, dass** das Signal eine Frequenz zwischen 0 Hz und 100 kHz hat und geeignet ist, die Moleküle der gasförmigen Arten (8) zu bewerten.

4. Verfahren gemäß Anspruch 2 oder 3, **dadurch gekennzeichnet, dass** das Signal eine Frequenz größer als 100 kHz hat, um auf die Bindung zwischen dem aktiven Material und den gasförmigen Sorten zu wechselwirken.

5. Verfahren gemäß einem der Ansprüche 2 bis 4, **dadurch gekennzeichnet, dass** das Signal eine sinusförmige Wellenform, eine quadratische Wellenform hat.

6. Verfahren gemäß einem der Ansprüche 2 bis 4, **dadurch gekennzeichnet, dass** das Signal eine pulsförmige Wellenform von beliebiger Form hat.

7. Verfahren gemäß einem der vorhergehenden Ansprüche, **dadurch gekennzeichnet, dass** das aktive Material nanostrukturierte Materialien umfasst, die zwischen den ersten (2) und den zweiten (3) Anschlüssen abgelagert sind, wobei die nanostrukturierten Materialien Nanoröhrchen umfassen.

8. Verfahren gemäß Anspruch 7, **dadurch gekennzeichnet, dass** die Nanoröhrchen Kohlenstoff-Nanoröhrchen (7) sind.

9. Verfahren gemäß Anspruch 8, **dadurch gekennzeichnet, dass** die Nanoröhrchen vom Einzelwand-Typ sind.

10. Verfahren gemäß einem der Ansprüche 7 bis 9, **dadurch gekennzeichnet, dass** die Nanoröhrchen in einer geordneten Weise, vorzugsweise durch Dielektrophorese abgelagert sind.

11. Verfahren gemäß einem der vorhergehenden Ansprüche, **dadurch gekennzeichnet, dass** das aktive Material Metalloxide umfasst, die zwischen den ersten und den zweiten Anschlüssen abgelagert sind.

12. Verfahren gemäß Anspruch 11, **dadurch gekennzeichnet, dass** die Metalloxide Zinnoxid ($SnO_2$) und/oder Wolframoxid ($WO_3$) umfassen.

13. Verfahren gemäß einem der vorhergehenden Ansprüche, **dadurch gekennzeichnet, dass** das aktive Material organische Halbleitermaterialen und organo-metallische Materialien umfasst, die zwischen den ersten (2) und den zweiten (3) Anschlüssen abgelagert sind.

14. Verfahren gemäß einem der vorhergehenden Ansprüche, **dadurch gekennzeichnet, dass** der Sensor eine isolierende Schicht (6) enthält, oberhalb derer die ersten (2) und die zweiten (3) Anschlüsse platziert sind, und unterhalb dessen der Kontroll-Anschluss platziert ist.

15. Verfahren gemäß Anspruch 14, **dadurch gekennzeichnet, dass** die isolierende Schicht (6) Siliziumdioxid ($SiO_2$) oder Glas umfasst.

16. Verfahren gemäß einem der vorhergehenden Ansprüche, **dadurch gekennzeichnet, dass** die ersten (2) und die zweiten (3) Anschluss ineinander greifen.

17. Verfahren gemäß einem der vorhergehenden Ansprüche, **dadurch gekennzeichnet, dass** die chemischen Substanzen Stickstoff-Dioxid $NO_2$ oder Ammoniak $NH_3$ umfassen.

## Revendications

1. Procédé de contrôle du temps de réponse d'un détecteur (1) de substances chimiques, ledit détecteur (1) étant composé d'une première (2) et d'une deuxième (3) broche ;
d'au moins une couche de matériau actif déposée entre ladite première (2) et ladite deuxième (3) broches, ledit matériau actif subissant une modification de sa propre impédance après contact avec lesdites substances chimiques et en fonction de leur concentration ; et
d'une autre broche de commande (4) ;
ledit procédé fournissant :

au moins une phase de détection desdites substances chimiques correspondant à une absorption desdites substances chimiques par ledit matériau, et
au moins une phase de récupération dudit détecteur (1), correspondant à une désorption desdites substances chimiques par ledit matériau, pendant lesdites phases de détection et de récupération, un signal étant appliqué à ladite broche de commande (4), adapté pour entraîner l'absorption ou la désorption dudit matériau, modifiant la durée des phases respectives de détection et de récupération ;
**caractérisé en ce que**
ledit signal fournit une composante de courant continu (CC) de façon :

- à polariser négativement ladite broche de commande (4) par rapport au matériau actif dans la phase d'absorption, dans le cas où la molécule du gaz à détecter permet une liaison avec ledit matériau actif de type donneur d'électrons, ou positivement dans le cas où la molécule du gaz à détecter permet une liaison avec ledit matériau actif de type enleveur d'électrons ;
- à polariser positivement ladite broche de commande (4) par rapport au matériau actif dans la phase de désorption, dans le cas où la molécule du gaz à détecter permet une liaison avec ledit matériau actif de type donneur d'électrons, ou négativement dans le cas où la molécule du gaz à détecter permet une liaison avec ledit matériau actif de type enleveur d'électrons.

2. Procédé selon la revendication 1, **caractérisé en ce que** ledit signal fournit une composante de fréquence.

3. Procédé selon la revendication 2, **caractérisé en ce que** ledit signal a une fréquence comprise entre 0 Hz et 100 KHz, convenant à la sollicitation des molécules desdites espèces gazeuses (8).

4. Procédé selon la revendication 2 ou 3, **caractérisé en ce que** ledit signal a une fréquence supérieure à 100 KHz, de façon à interagir sur la liaison parmi ledit matériau actif et lesdites espèces gazeuses.

5. Procédé selon l'une des revendications 2 à 4, **caractérisé en ce que** ledit signal a une forme d'onde sinusoïdale, une forme d'onde quadratique.

6. Procédé selon l'une des revendications 2 à 4, **caractérisé en ce que** ledit signal a une forme d'onde pulsée de forme arbitraire.

7. Procédé selon l'une des revendications précédentes, **caractérisé en ce que** ledit matériau actif est composé de matériaux nanostructurés déposés entre ladite première (2) et ladite deuxième (3) broches, lesdits matériaux nanostructurés comprenant des nanotubes.

8. Procédé selon la revendication 7, **caractérisé en ce que** lesdits nanotubes sont des nanotubes de carbone (7).

9. Procédé selon la revendication 8, **caractérisé en ce que** lesdits nanotubes sont à paroi unique.

10. Procédé selon l'une des revendications 7 à 9, **caractérisé en ce que** lesdits nanotubes sont déposés de manière ordonnée, de préférence par diélectrophorèse.

11. Procédé selon l'une des revendications précédentes, **caractérisé en ce que** ledit matériau actif est composé d'oxydes métalliques déposés entre ladite première et ladite deuxième broches.

**12.** Procédé selon la revendication 11, **caractérisé en ce que** lesdits oxydes métalliques sont l'oxyde d'étain ($SnO_2$) et/ou l'oxyde de tungstène ($WO_3$).

**13.** Procédé selon l'une des revendications précédentes, **caractérisé en ce que** ledit matériau actif est composé de matériaux semi-conducteurs organiques et de matériaux organométalliques déposés entre ladite première (2) et ladite deuxième (3) broches.

**14.** Procédé selon l'une des revendications précédentes, **caractérisé en ce que** ledit détecteur comprend une couche isolante (6), au-dessus de laquelle ladite première (2) et ladite deuxième (3) broches sont placées, et sous laquelle ladite broche de commande est placée.

**15.** Procédé selon la revendication 14, **caractérisé en ce que** ladite couche isolante (6) est composée de dioxyde de silicium ($SiO_2$) ou de verre.

**16.** Procédé selon l'une des revendications précédentes, **caractérisé en ce que** ladite première (2) et ladite deuxième (3) broches sont interdigitées.

**17.** Procédé selon l'une des revendications précédentes, **caractérisé en ce que** lesdites substances chimiques comprennent du dioxyde d'azote $NO_2$ ou de l'ammoniac $NH_3$.

Fig. 1

Fig. 2

EP 1 831 681 B1

**Fig. 3**

**Fig. 4**

**Fig. 5**

**Fig. 6**

**Fig. 7**

Fig. 8

Fig. 9

**Fig. 10a**

**Fig. 10b**

Sensor with Ordered SWCNT, Gate OV

**Fig. 11a**

**Fig. 11b**

Gate 0V, T=23°c

EP 1 831 681 B1

**Fig. 12**

**Fig. 13**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 4442396 A1
- EP 425119 A1